# DEMANDE DE BREVET EUROPEEN

(11) **EP 1 702 610 A1**
(43) Date de publication de la demande: **20.09.2006**
(21) Numéro de dépôt: 06110283.6
(22) Date de dépôt: 22.02.2006
(51) Int. Cl.: A61K 8/42, A61K 8/365, A61Q 5/02, A61Q 5/12, A61Q 19/10

(54) **Composition cosmétique de nettoyage contenant un composé d'urée et un composé alkyl glycol carboxylate**

(30) Priorité: 17.03.2005 FR 0550684
(71) Demandeur: L'Oreal-D.I.P.I., 75008 Paris (FR)
(72) Inventeur: Aubrun-Sonneville, Odile, 92160, ANTONY (FR)
(74) Mandataire: Rasson, Catherine

(57) **Abrégé**

L'invention se rapporte à une composition cosmétique de nettoyage contenant dans un milieu aqueux, (1) au moins un tensioactif anionique choisi parmi les acides alkyl glycol carboxyliques et leurs sels, et (2) au moins un composé de formule (I) suivante : 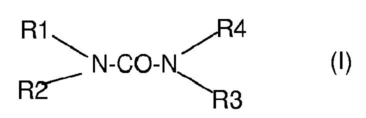 dans laquelle :
R1, R2, R3 et R4 représentent chacun, indépendamment l'un de l'autre, un atome d'hydrogène, un groupe alkyle en C1-C4 ou un groupe hydroxyalkyle en C2-C6 pouvant contenir de 1 à 5 groupes hydroxyles, où au moins un des radicaux R1 à R4 représente un groupe hydroxyalkyle,
ainsi que leurs sels, leurs solvats, et leurs isomères.

Le composé de formule (I) est de préférence l'hydroxyéthylurée.

L'invention se rapporte aussi à l'utilisation de la dite composition comme produit de nettoyage et/ou de démaquillage de la peau, comme produit de douche, comme shampoing ou après-shampoing, comme produit de rasage, comme produit exfoliant.

## Description

L'invention se rapporte à une composition cosmétique de nettoyage contenant dans un milieu aqueux, un composé d'urée hydroxylé et un tensioactif anionique particulier, et à ses utilisations pour le nettoyage et/ou le démaquillage de la peau humaine, des lèvres, et/ou des cheveux.

Le nettoyage de la peau est très important pour le soin du visage, et il doit être le plus performant possible car les résidus gras tels que l'excès de sébum, les restes des produits cosmétiques utilisés quotidiennement et les produits de maquillage s'accumulent dans les replis cutanés et peuvent obstruer les pores de la peau et entraîner l'apparition de boutons. Un moyen pour bien nettoyer la peau est d'utiliser des produits de nettoyage moussants. On cherche en général à disposer de produits donnant un volume de mousse important, signe d'une bonne efficacité nettoyante, et certains tensioactifs comme les alkyls glycols carboxylates sont connus pour donner des mousses de bonne qualité.

On cherche également à avoir des produits confortables, qui ne laissent pas de sensation de tiraillements ou de sécheresse. En effet, une des difficultés majeures de la formulation de produits moussants consiste à mettre au point des produits ayant un très bon pouvoir moussant pour assurer un bon nettoyage et pouvant se rincer facilement, tout en respectant la barrière cutanée, c'est-à-dire sans dessécher la peau.

Un moyen d'améliorer la sensation de confort et d'hydratation est d'ajouter des agents hydratants comme la glycérine. Cependant, ce type de dérivé a tendance à altérer les performances moussantes des produits, en particulier en diminuant le volume de mousse obtenu.

Il subsiste donc le besoin de disposer de compositions cosmétiques de nettoyage, qui ne soient pas desséchantes pour la peau et qui aient néanmoins un bon pouvoir moussant et une bonne rincabilité.

La demanderesse a découvert de manière surprenante qu'il est possible d'obtenir des produits nettoyants, apportant un effet hydratant, sans compromettre la qualité de mousse, en particulier sans altérer le volume de mousse, en associant au moins un dérivé d'alkyl glycol carboxylate avec un dérivé d'urée.

Ainsi, l'invention a pour objet une composition cosmétique de nettoyage contenant dans un milieu aqueux, (1) au moins un tensioactif anionique choisi parmi les acides alkyl glycol carboxyliques et leurs sels, et (2) au moins un composé de formule (I) suivante : 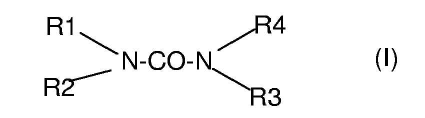 dans laquelle :
R1, R2, R3 et R4 représentent chacun, indépendamment l'un de l'autre, un atome d'hydrogène, un groupe alkyle en C1-C4 ou un groupe hydroxyalkyle en C2-C6 pouvant contenir de 1 à 5 groupes hydroxyles, où au moins un des radicaux R1 à R4 représente un groupe hydroxyalkyle, ainsi que leurs sels, leurs solvats, et leurs isomères.

La composition de l'invention présente l'avantage de ne pas dessécher la peau tout en ayant un bon pouvoir moussant et tout en étant bien rinçable. Le fait d'utiliser comme tensioactif, un acide alkyl glycol carboxylique ou un de ses sels, en association avec un composé de formule (I) comme hydratant, permet d'avoir un produit donnant une mousse de très bonne qualité tout en ayant un bon pouvoir hydratant. On n'obtient pas un tel résultat en absence de tensioactif du type acide alkyl glycol carboxylique.

On entend ici par « milieu aqueux », un milieu contenant de l'eau et éventuellement un ou plusieurs solvants organiques hydrosolubles. Dans ce milieu aqueux, la quantité d'eau est de préférence d'au moins 20 % en poids, et elle va de préférence de 20 à 95 % en poids, mieux de 30 à 90 % en poids et encore mieux de 40 à 85 % en poids par rapport au poids total de la composition.

En outre, la composition de l'invention étant une composition cosmétique, elle contient un milieu physiologiquement acceptable. On entend ici par « milieu physiologiquement acceptable », un milieu compatible avec la peau, les muqueuses, les yeux. Il s'agit de préférence d'un milieu cosmétiquement acceptable, c'est-à-dire qui, en outre, présente une couleur, une odeur et un toucher agréables et qui ne génère pas d'inconforts inacceptables (picotements, tiraillements, rougeurs), susceptibles de détourner la consommatrice d'utiliser cette composition.

De façon avantageuse, le pH du milieu aqueux est compatible avec les matières kératiniques et notamment avec la peau. Ce pH va de préférence de 3 à 8,5, mieux de 3,5 à 8, préférentiellement de 5 à 7,5.

La composition de nettoyage de l'invention est une composition moussante qui est rincée après application sur la peau.

### Composé d'urée hydroxylé

Le composé d'urée présent dans la composition selon l'invention est un composé de formule (I) suivante : 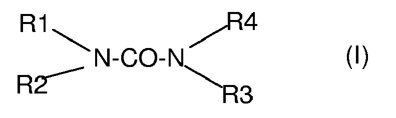 dans laquelle :
R1, R2, R3 et R4 représentent chacun, indépendamment l'un de l'autre, un atome d'hydrogène, un groupe alkyle en C1-C4 ou un groupe hydroxyalkyle en C2-C6 pouvant contenir de 1 à 5 groupes hydroxyles, où au moins un des radicaux R1 à R4 représente un groupe hydroxyalkyle,
   ainsi que leurs sels, leurs solvats, et leurs isomères.

Dans les composés de formule (I) :
- De préférence R1 désigne un groupe hydroxyalkyle en C2-C6 et R2, R3, R4 désignent, indépendamment l'un de l'autre, un atome d'hydrogène ou un groupe alkyle en C1-C4 ;
- Préférentiellement R1 désigne un groupe hydroxyalkyle en C2-C6 comprenant de 1 à 5 groupes hydroxyles, notamment 1 groupe hydroxyle, et R2, R3, R4 désignent un atome d'hydrogène ;
- Plus préférentiellement, R1 désigne un groupe hydroxyalkyle en C2-C4 comprenant 1 groupe hydroxyle et R2, R3, R4 désignent un atome d'hydrogène.

Comme groupes alkyle en C1-C4, on peut citer notamment les groupes méthyle, éthyle, n-propyle, isopropyle, n-butyle, iso-butyle et tert-butyle.

Parmi les groupes hydroxyalkyle en C1-C6, on préfère ceux contenant un seul groupe hydroxyle et en particulier les groupes hydroxyéthyle, hydroxypropyle, hydroxybutyle, hydroxypentyle et hydroxyhexyle.

Comme sels de tels composés, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, acide sulfonique ou acide phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Par solvat, on entend un mélange stoechiométrique dudit composé de formule (I) avec une ou plusieurs molécules d'eau ou de solvant organique, un tel mélange étant issu de la synthèse du composé de formule (I).

Comme composés de formule (I) préférés, on peut citer le N-(2-hydroxyéthyl)-urée ; le N-(2-hydroxypropyl)-urée ; le N-(3-hydroxypropyl)-urée ; le N-(2,3-dihydroxypropyl)-urée ; le N-(2,3,4,5,6-pentahydroxyhexyl)-urée ; le N-méthyl-N-(1,3,4,5,6-pentahydroxy-2-hexyl)-urée ; le N-méthyl-N'-(1-hydroxy-2-méthyl-2-propyl)-urée ; le N-(1-hydroxy-2-méthyl-2-propyl)-urée ; le N-(1,3-dihydroxy-2-propyl)-urée ; le N-(tris-hydroxyméthyl-méthyl)-urée ; le N-éthyl-N'-(2-hydroxyéthyl)-urée ; le N,N-bis-(2-hydroxyéthyl)-urée ; le N,N'-bis-(2-hydroxyéthyl)-urée ; le N,N-bis-(2-hydroxypropyl)-urée ; le N,N'-Bis-(2-hydroxypropyl)-urée ; le N,N-Bis-(2-hydroxyéthyl)-N'-propyl-urée ; le N,N-Bis-(2-hydroxypropyl)-N'-(2-hydroxyéthyl)-urée ; le N-tert.Butyl-N'-(2-(hydroxyéthyl)-N'-(2-(hydroxypropyl)-urée; le N-(1,3-dihydroxy-2-propyl)-N'-(2-hydroxyéthyl)-urée ; le N,N-Bis-(2-hydroxyéthyl)-N',N'-dimethyl-urée ; le N,N,N',N'-tetrakis-(2-hydroxyéthyl)-urée ; le N',N'-Bis-(2-hydroxyéthyl)-N',N'-bis-(2-hydroxypropyl)-urée ; et leurs mélanges.

De préférence, le composé de formule (I) est le N-(2-hydroxyéthyl)-urée.

Les composés de formule (I) sont des composés connus et notamment décrits dans la demande DE-A-2703185. Parmi ceux-ci, le N-(2-hydroxyéthyl)-urée est en outre disponible dans le commerce, sous forme de mélange à 50% en poids dans l'eau, auprès de la société National Starch sous la dénomination commerciale Hydrovance® .

Le composé de formule (I) peut être présent dans la composition selon l'invention en une quantité (en matière active) allant de 0,1 % à 50 % en poids, par rapport au poids total de la composition, de préférence allant de 0,1 % à 20 % en poids, et préférentiellement allant de 0,1 % à 10 % en poids.

### Tensioactif anionique

La composition selon l'invention comprend au moins un tensioactif anionique choisi parmi les acides alkyl glycol carboxyliques (ou acides 2-(2-Hydroxyalkyloxy acétique), et leurs sels. Ces tensioactifs sont des tensioactifs moussants.

Ces tensioactifs peuvent notamment choisis parmi les composés de formule (II) suivante :

R₁-CHOH-CH₂-O-COO⁻ X⁺ (II)

dans laquelle R₁ désigne un radical alkyle saturé ou insaturé, linéaire ou ramifié, comportant de 8 à 30 atomes de carbone, et X désigne l'hydrogène ou un cation minéral ou organique tel que ceux issus d'un métal alcalin (par exemple Na+, K+), NH4+, les ammoniums issus des aminoacides basiques tels que la lysine, l'arginine, la sarcosine, l'ornithine, la citrulline ou bien encore des amino-alcools tels que la monoéthanolamine, la diéthanolamine, la triéthanolamine, la glucamine, la N-méthyl glucamine, l'amino-3 propanediol-1,2.

Les acides hydroxy-2 alkyl carboxyliques préférés selon la présente invention sont des composés de formule (II) dans laquelle R₁ désigne plus particulièrement un radical alkyle saturé ou insaturé, linéaire ou ramifié comportant de 8 à 18 atomes de carbone.

Parmi les tensioactifs de formule (II), on peut citer notamment le lauryl glycol carboxylate de sodium, commercialisé sous les dénominations BEAULIGHT SHAA® ou BEAULIGHT LCA-25N® par la société SANYO, ou sa forme acide correspondante commercialisée sous la dénomination BEAULIGHT SHAA (Acid Form)® par la société SANYO.

La quantité (en matière active) de tensioactifs anioniques de type alkyl glycol carboxylique peut aller par exemple de 0,1 à 50 % en poids, de préférence de 0,5 à 20 % en poids et mieux de 1 à 15 % en poids par rapport au poids total de la composition.

### Autres tensioactifs moussants

La composition peut contenir un ou plusieurs autres tensioactifs moussants qui peuvent être choisis parmi les tensioactifs moussants non-ioniques, amphotères ou zwitterioniques, anioniques. La quantité (en matière active) de ces autres tensioactifs moussants peut aller par exemple de 0,1 à 20 % en poids et mieux de 0,5 à 15 % en poids par rapport au poids total de la composition.

Comme tensioactifs non ioniques moussants, la composition peut contenir par exemple, un ou plusieurs tensioactifs non ioniques choisis parmi les alkyl polyglucosides (APG), les esters de maltose, les alcools gras polyglycérolés, les dérivés de glucamine comme l'éthyl-2 hexyl oxy-carbonyl n-méthyl glucamine, et leurs mélanges.

Comme alkylpolyglucosides, on utilise de préférence ceux contenant un groupe alkyle comportant de 6 à 30 atomes de carbone et de préférence de 8 à 16 atomes de carbone, et contenant un groupe hydrophile (glucoside) comprenant de préférence 1,2 à 3 unités de glucoside. Comme alkylpolyglucosides, on peut citer par exemple le decylglucoside (Alkyl-C9/C11-polyglucoside (1.4)) comme le produit commercialisé sous la dénomination MYDOL 10® par la société Kao Chemicals, le produit commercialisé sous la dénomination PLANTAREN 2000 UP® par la société Cognis, et le produit commercialisé sous la dénomination ORAMIX NS 10 ® par la société Seppic ; le caprylyl/capryl glucoside comme le produit commercialisé sous la dénomination ORAMIX CG 110® par la Société Seppic ; le laurylglucoside comme les produits commercialisés sous les dénominations PLANTAREN 1200 N® et PLANTACARE 1200® par la société Cognis ; le coco-glucoside comme le produit commercialisé sous la dénomination PLANTACARE 818/UP® par la société Cognis ; et leurs mélanges.

Les dérivés de maltose sont par exemple ceux décrits dans le document EP-A-566438, tels que l'O-octanoyl-6'-D-maltose, ou encore le O-dodecanoyl-6'-D-maltose décrit dans le document FR-A-2,739,556.

Comme alcools gras polyglycérolés, on peut citer par exemple le dodecanediol polyglycérolé (3,5 moles de glycérol), produit commercialisé sous la dénomination CHIMEXANE NF® par la société Chimex.

Les tensioactifs moussants amphotères et zwitterioniques peuvent être choisis par exemple parmi les dérivés de bétaïnes dont les amidopropylbétaïnes, les amphoacétates et amphodiacétates, les hydroxylsultaines et leurs mélanges.

Comme dérivés de bétaïnes, on peut citer par exemple la cocobétaïne comme le produit commercialisé sous la dénomination DEHYTON AB-30® par la société Cognis ; la laurylbétaïne comme le produit commercialisé sous la dénomination GENAGEN KB® par la société Clariant ; la laurylbétaïne oxyethylénée (10 OE), comme le produit commercialisé sous la dénomination LAURYLETHER(10 OE)BETAINE® par la société Shin Nihon Rica ; la stéarylbétaïne oxyéthylénée (10 OE) comme le produit commercialisé sous la dénomination STEARYLETHER(10 OE)BETAINE® par la société Shin Nihon Rica ; la cocamidopropyl betaine commercialisé par exemple sous la dénomination VELVETEX BK 35® par la société Cognis ; le undecylenamidopropyl betaïne commercialisé par exemple sous la dénomination AMPHORAM U® par la société Ceca ; et leurs mélanges.

Comme amphoacétates et amphodiacétates, on peut citer par exemple le N-cocoyl-N-carboxyméthoxyéthyl-N-carboxyméthyl-éthylènediamine N-di-sodique (nom CTFA: disodium cocamphodiacetate) comme le produit commercialisé sous la dénomination MIRANOL C2M CONCENTRE NP® par la société Rhodia Chimie ; le N-cocoyl-N-hydroxyéthyl-N-carboxyméthyl-éthylènediamine N-sodique (nom CTFA : sodium cocamphoacetate) et leurs mélanges.

Les tensioactifs anioniques peuvent être choisis par exemple parmi les savons (sel alcalins d'acides gras), les acylaminoacides tels que les acylglycinates, les amidoéther carboxylates, les alkyl polyaminocarboxylates, les alkyl éthers sulfates tels que les Sodium Laureth sulfates, les alkyl sulfonates, les iséthionates, les alkyl méthyltaurates, les alkyl sulfosuccinates, les alkyl sulfoacétates, les monoalkylphosphates, les dialkylphosphates, leurs sels, et leurs mélanges.

### Adjuvants

Comme indiqué ci-dessus, le milieu aqueux de la composition selon l'invention peut contenir, outre l'eau, un ou plusieurs solvants hydrosolubles qui peuvent être choisis par exemple parmi les alcools inférieurs (mono-alcools) comportant de 1 à 6 atomes de carbone, tels que l'éthanol ; et les polyols tels que la glycérine, les glycols comme le butylène glycol, l'isoprène glycol, le propylène glycol, les polyéthylène glycols comme le PEG-8 ; le sorbitol ; les sucres tels que le glucose, le fructose, le maltose, le lactose, le sucrose ; et leurs mélanges, dans la mesure où ces composés n'altèrent pas les propriété recherchées de la composition selon l'invention. La quantité de solvant(s) dans la composition de l'invention peut aller par exemple de 0,1 à 15 % en poids, de préférence de 0,5 à 10 % en poids, mieux de 0,5 à 7,5 % en poids et encore mieux de 0,5 à 5 % en poids par rapport au poids total de la composition.

La composition selon l'invention peut aussi contenir tout adjuvant ou additif habituellement utilisé dans le domaine cosmétique. Bien entendu, l'homme de l'art veillera à choisir le ou les éventuels additifs de la composition selon l'invention de manière telle que les propriétés avantageuses attachées intrinsèquement à la composition conforme à l'invention ne soient pas ou substantiellement pas, altérées par l'addition envisagée.

Parmi les adjuvants classiques susceptibles d'être ajoutés, on peut citer les conservateurs ; les séquestrants (EDTA) ; les antioxydants ; les parfums ; les matières colorantes telles que les colorants solubles, les pigments et les nacres ; les charges à effets matifiant, tenseur, blanchissant ou exfoliant ; les filtres solaires ; les actifs cosmétiques ou dermatologiques, hydrophiles ou lipophiles, tels que les vitamines, les antiseptiques, les antisébohrréïques, les antimicrobiens comme le peroxyde de benzoyle, l'acide salicylique, le triclosan, l'acide azélaïque, la niacine (vit. PP), les amincissants comme la caféine, les azurants optiques, les électrolytes, les agents ayant pour effet d'améliorer les propriétés cosmétiques de la peau ou encore des particules solides sphériques ou non, poreuses ou non de toute taille. Les quantités de ces différents adjuvants sont celles classiquement utilisées dans le domaine considéré, et par exemple de 0,01 à 20 % du poids total de la composition. Ces adjuvants ainsi que leurs concentrations doivent être tels qu'ils ne modifient pas la propriété recherchée pour la composition de l'invention.

Comme exfoliants, on peut citer par exemple des particules exfoliantes ou gommantes d'origine minérale, végétale ou organique. Ainsi, on peut utiliser par exemple des billes ou de la poudre de polyéthylène, de la poudre de nylon, de la poudre de polychlorure de vinyle, de la pierre ponce, des broyats de noyaux d'abricots ou de coques de noix, de la sciure de bois, des billes de verre, l'alumine, et leurs mélanges. Ces particules peuvent être présentes en une quantité allant par exemple de 0,5 à 30 % en poids, de préférence de 1 à 20 % en poids et mieux de 1 à 10 % en poids par rapport au poids total de la composition. Quand la composition contient des particules exfoliantes, elle peut constituer notamment une composition de gommage de la peau du visage ou du corps.

Les compositions peuvent contenir également des polymères hydrophiles ou lipophiles, anioniques, non ioniques, cationiques ou amphotères, épaississants ou dispersants, dans la mesure où ils n'affectent pas les qualités de la composition.

Comme polymères cationiques ou amphotères, on peut citer notamment ceux du type Polyquaternium (nom CTFA), qui apportent douceur et onctuosité à la crème moussante. Ces polymères peuvent être choisis de préférence parmi les polymères suivants :
- Polyquaternium 5 tel que le produit MERQUAT 5 commercialisé par la société CALGON ;
- Polyquaternium 6 tel que le produit SALCARE SC 30 commercialisé par la société CIBA, et le produit MERQUAT 100 commercialisé par la société CALGON ;
- Polyquaternium 7 tel que les produits MERQUAT S, MERQUAT 2200 et MERQUAT 550 commercialisés par la société CALGON, et le produit SALCARE SC 10 commercialisé par la société CIBA ;
- Polyquaternium 10 tel que le produit Polymer JR400 commercialisé par la société AMERCHOL ;
- Polyquaternium 11 tel que les produits GAFQUAT 755, GAFQUAT 755N et GAFQUAT 734 commercialisés par la société ISP ;
- Polyquaternium 15 tel que le produit ROHAGIT KF 720 F commercialisé par la société ROHM ;
- Polyquaternium 16 tel que les produits LUVIQUAT FC905, LUVIQUAT FC370, LUVIQUAT HM552 et LUVIQUAT FC550 commercialisés par la société BASF ;
- Polyquaternium 22 tel que le produit MERQUAT 280 commercialisé par la société CALGON ;
- Polyquaternium 28 tel que le produit STYLEZE CC10 commercialisé par la société ISP ;
- Polyquaternium 39 tel que le produit MERQUAT PLUS 3330 commercialisé par la société CALGON ;
- Polyquaternium 44 tel que le produit LUVIQUAT CARE commercialisé par la société BASF ;
- Polyquaternium 46 tel que le produit LUVIQUAT HOLD commercialisé par la société BASF ;
- Polyquaternium 47 tel que le produit MERQUAT 2001 commercialisé par la société CALGON.

On peut aussi utiliser comme polymère cationique, les guars cationiques telles que les produits JAGUAR commercialisés par la société RHODIA.

Comme polymères anioniques, on peut citer notamment ceux comportant au moins une chaîne hydrophobe, et en particulier ceux dérivés d'acide acrylique ou méthacrylique, comme le copolymère acrylates/steareth-20 methacrylate commercialisé sous la dénomination Aculyn 22 par la société Rohm & Haas (nom CTFA : Acrylates/Steareth-30 Methacrylate copolymer) ; le terpolymère acide (méth)acrylique / acrylate d'éthyle / méthacrylate de béhényle oxyéthyléné (25 OE), en émulsion aqueuse commercialisé sous la dénomination Aculyn 28 par la société Rohm & Haas ; le copolymère acide acrylique/itaconate de mono-cétyle oxyéthyléné (20 OE), en dispersion aqueuse à 30 % commercialisé sous la dénomination Structure 3001 par la société National Starch ; le copolymère acide acrylique/itaconate de mono-stéaryle oxyéthyléné (20 OE) en dispersion aqueuse à 30 % commercialisé sous la dénomination Structure 2001 par la société National Starch ; le copolymère acrylates/acrylate modifié par des alcools en C12-C24 polyoxyéthylénés (25 OE), sous forme de latex à 30-32 % de copolymère, commercialisé sous la dénomination Synthalen W2000 par la société 3V SA, ainsi que les copolymères vendus sous les noms PEMULEN ou CARBOPOL par la Société Noveon, comme le copolymère acrylate/C₁₀-C₃₀-alkylacrylate tel que les produits PEMULEN TR1, PEMULEN TR2 ou CARBOPOL 1382 (nom CTFA : Acrylates/C10-30 Alkyl acrylate Crosspolymer).

La composition peut contenir aussi des polymères comportant au moins un monomère à groupement sulfonique, et notamment les polymères et copolymères d'acide 2-acrylamido-2-méthylpropane sulfonique (AMPS) tels que :
- l'homopolymère réticulé et neutralisé d'acide 2-acrylamido 2-méthylpropane sulfonique, commercialisé par la société Clariant sous la dénomination commerciale « Hostacerin AMPS » (nom CTFA : ammonium polyacryldimethyltauramide) ;
- les copolymères anioniques réticulés d'acrylamide ou méthylacrylamide et d'acide 2-acrylamido-2-méthylpropane sulfonique, notamment ceux se présentant sous la forme d'une émulsion E/H, tels que ceux commercialisés sous le nom de SEPIGEL 305 par la société Seppic (nom C.T.F.A. : Polyacrylamide / C13-14 Isoparaffin / Laureth-7), sous le nom de SIMULGEL 600 par la société Seppic (nom C.T.F.A. : Acrylamide / Sodium acryloyldimethyltaurate copolymer /Isohexadecane / Polysorbate 80),
- les copolymères d'acide(méth)acrylique ou de (méth)acrylate et d'acide 2-acrylamido- 2-méthylpropane sulfonique, notamment ceux se présentant sous la forme d'une émulsion E/H, tels que ceux commercialisés sous le nom de SIMULGEL NS par la société Seppic (copolymère d'acrylamido-2-methyl propane sulfonate de sodium / hydroxyethyl acrylate en émulsion inverse à 40% dans Polysorbate 60 et squalane) (nom CTFA : hydroxyethyl acrylate/sodium acryloyldimethyltaurate copolymer / squalane / polysorbate 60) ou ceux commercialisés sous le nom de SIMULGEL EG par la société Seppic (copolymère d'acide acrylique / acrylamido-2-methylpropane-sulfonique sous forme de sel de sodium en émulsion inverse à 45% dans isohexadecane / eau) (nom C.T.F.A. : Sodium Acrylate / Sodium acryloydimethyl-taurate copolymer / Isohexadecane /Polysorbate 80),
- les copolymères d'acide 2-acrylamido-2-méthylpropane sulfonique et de vinylpyrrolidone ou de vinylformamide, tels que les produits commercialisés sous les dénominations ARISTOFLEX AVC par la société Clariant,
- les polymères d'AMPS modifiés hydrophobes comme notamment le copolymère d'AMPS et de methacrylate d'alcool en C12-C14 éthoxylé (copolymère non réticulé obtenu à partir de Genapol LA-070 et d'AMPS) (nom CTFA : Ammonium Acryloyldimethyltaurate / Laureth-7 Methacrylate Copolymer) commercialisé sous la dénomination ARISTOFLEX LNC par la société Clariant, et le copolymère d'AMPS et de méthacrylate de stéaryle éthoxylé (25 EO) (copolymère réticulé par le trimethylolpropane triacrylate, obtenu à partir de Genapol T-250 et d'AMPS) (nom CTFA : Ammonium Acryloyldimethyltaurate / Steareth-25 Methacrylate Crosspolymer) commercialisé sous la dénomination ARISTOFLEX HMS par la société Clariant.

Ces polymères peuvent être en une quantité (en matière active) allant par exemple de 0,05 à 10 % en poids et mieux de 0,1 à 5 % en poids par rapport au poids total de la composition.

Les compositions selon l'invention se présentent sous forme de solutions, de gels, de laits ou de crèmes plus ou moins souples, et elles peuvent être utilisées sur toutes les matières kératiniques telles que la peau, le cuir chevelu, les cheveux, les cils, les sourcils, les ongles ou les muqueuses, notamment comme produit d'hygiène, par exemple comme produit de nettoyage de la peau, des muqueuses et/ou des cheveux, en particulier comme produit de nettoyage et/ou de démaquillage de la peau (du visage et/ou du corps), comme produit de douche (produit deux-en-un), comme shampoing, comme après-shampoing, comme produit de rasage, comme masque à rincer, comme produit exfoliant (appelé aussi desquamant ou désincrustant) aussi bien pour le visage que pour le corps ou pour les mains, après addition de particules exfoliantes.

L'invention a encore pour objet l'utilisation cosmétique de la composition telle que définie ci-dessus, comme produit de nettoyage et/ou de démaquillage de la peau, comme produit de douche, comme shampoing ou après-shampoing, comme produit de rasage, comme masque à rincer, comme produit exfoliant.

Un autre objet de l'invention est un procédé de nettoyage d'une matière kératinique, telle que la peau, du cuir chevelu, des cheveux, des cils, des sourcils, des ongles ou des muqueuses, caractérisé par le fait qu'on applique sur la matière kératinique, une composition telle que définie ci-dessus et qu'on la rince.

La matière kératinique est de préférence la peau.

Les compositions selon l'invention peuvent être par exemple utilisées de la manière suivante :
1) quand les compositions selon l'invention constituent des produits de nettoyage doux pour le visage, elles peuvent être utilisées de la manière suivante :
   - on fait mousser le produit dans les mains avec de l'eau
   - on applique la mousse sur le visage
   - on nettoie le visage
   - on rince à l'eau
2) quand les compositions selon l'invention constituent des produits démaquillants, elles peuvent être utilisées comme indiqué ci-dessus mais elles peuvent aussi être appliquées à sec sur le visage, puis massées jusqu'à obtention d'un démaquillage satisfaisant, et rincées à l'eau, ou bien elles peuvent être appliquées au moyen d'un coton.
3) on peut les utiliser comme produit de douche deux-en-un, comme shampooing et/ou après shampooing, comme masque à rincer ou comme produit de rasage, de la manière habituelle d'utilisation de ces produits.

Les exemples qui suivent servent à illustrer l'invention sans toutefois présenter un caractère limitatif. Les quantités indiquées correspondent, sauf mention contraire, à la quantité de matière active et non à la quantité de matière première. Les noms des composés utilisés sont indiqués en nom CTFA ou en nom chimique.

### Exemple 1 selon l'invention et exemples comparatifs 1 et 2

| Composition moussante | Exemple 1 selon l'invention | Exemple comparatif 1 | Exemple comparatif 2 |
|---|---|---|---|
| Eau | Qsp 100 % | Qsp 100 % | Qsp 100 % |
| N-(2-hydroxyéthyl)-urée (1) | 10 % | - | 10 % |
| Sodium lauryl glycol carboxylate. (2) | 10,4 % | 10,4 % | - |
| Sodium laureth sulfate | - | - | 10,4 % |
| Glycérine | - | 10% | - |
| Acide citrique | 0,1 % | 0,1 % | 0,1% |
| Conservateurs | 0,4 ù | 0,4 % | 0,4 % |
| pH | 7 | 7 | 7,4 |
| Volume de mousse 1 | 4,5 | 3;2 | 2,3 |
| Volume de mousse 2 | 8,3 | 6,7 | 5 |

| | | | |
|---|---|---|---|
| (1) soit 20 % d'Hydrovance commercialisé par la société National Starch (Hydroxyéthyl urée à 50% en poids en solution aqueuse) (2) Beaulight Shaa commercialisé par la société Sanyo | | | |

Mode opératoire : on prépare une solution aqueuse contenant l'agent hydratant (hydrovance ou glycérine), le tensioactif (Beaulight ou Laureth sulfate) et les conservateurs, puis on ajoute l'acide citrique, et la quantité d'eau est ajustée à 100%.

### Test de performance des compositions :

Les qualités de mousse des compositions des exemples indiqués ci-dessus ont été évaluées selon le protocole suivant :

Avant toute utilisation des produits, les mains sont lavées au savon de Marseille puis convenablement rincées et séchées.
1- mouiller les mains en les passant sous l'eau courante, les secouer trois fois pour les essorer
2- placer 1 g de produit dans le creux de l'une des mains
3- travailler le produit entre les deux paumes pendant 10 secondes,
4- évaluer le volume de mousse (volume de mousse 1)
5- ajouter 2ml d'eau et travailler le produit à nouveau pendant 10 secondes,
6- évaluer le volume de mousse (volume de mousse 2)

Le volume est noté sur une échelle de 0 à 10.

Le panel d'évaluation est constitué de 3 experts entraînés. La moyenne des trois notes permet de comparer les produits selon chacun des critères.

Les résultats de volume de mousse 1 et de volume de mousse 2 sont indiqués dans le tableau ci-dessus. Ils montrent que l'exemple selon l'invention assure une bonne hydratation de la peau tout en donnant un meilleur volume de mousse que l'exemple comparatif 1 qui contient de la glycérine au lieu de l'hydrovance, et que l'exemple comparatif 2 qui contient un autre tensioactif anionique que le Beaulight.

### Exemples 2 à 5 selon l'invention

| Composition | Exemple 2 | Exemple 3 | Exemple 4 | Exemple 5 |
|---|---|---|---|---|
| Sodium Lauryl Glycol Carboxylate (1) | 7.8 | 3.9 | 7.8 | 3.9 |
| Coco-bétaine (2) | 6.5 | 3.25 | - | - |
| Cocamidopropyl bétaine (3) | - | - | 6.5 | - |
| Coco-Glucoside (4) | - | - | - | 6.5 |
| Ammonium Acryloyldimethyltaurate / Steareth-25 Methacrylate Crosspolymer (5) | - | 1 | - | - |
| PEG-150 pentaérythrityle Tétrastéarate (6) | 1 | - | 2 | - |
| Acrylates/C10-30 Alkyl acrylate Crosspolymer (7) | - | - | - | 1 |
| Silice (8) | - | - | 5 | - |
| Polyquaternium-7 | 0.3 | - | - | - |
| PEG-14M (9) | - | - | - | 0.5 |
| N-(2-hydroxyéthyl)-urée (10) | 10 | 5 | 10 | 10 |
| Acide citrique | Qs pH7 | Qs pH7 | Qs pH7 | Qs pH7 |
| DMDM Hydantoin | 0.2 | 0.2 | 0.2 | 0.2 |
| Triéthanolamine | - | - | - | 1 |
| Sodium Méthylparaben | 0.3 | 0.3 | 0.3 | 0.3 |
| Eau | Qsp100 | Qsp100 | Qsp100 | Qsp100 |

| | | | | |
|---|---|---|---|---|
| (1) Beaulight Shaa commercialisé par la société Sanyo (2) Dehyton AB 30® commercialisé par la société Cognis, (contenant 30% en poids de matière active) (3) Tegobetain F50® commercialisé par la société Goldschmidt (à 38% en matière active) (4) Plantacare 818 UP commercialisé par la société Cognis (à 53% en matière active) (5) Aristoflex HMS commercialisé par la société Clariant (6) CROTHIX commercialisé par la société Croda. (7) Carbopol 1382 commercialisé par la société Noveon (8) Aerosil 200 commercialisé par la société Deguss-Hüls (9) POLYOX WSR 205 commercialisé par la société Amerchol. (10) Hydrovance commercialisé par la société National Starch (à 50% de matière active en solution aqueuse) | | | | |

### Exemples 6 et 7 selon l'invention

| Composition | Exemple 6 | Exemple 7 |
|---|---|---|
| Sodium Lauryl Glycol Carboxylate (1) | 7.8 | 7.8 |
| Sodium lauryl éther sulfate | 3.25 | - |
| Potassium Cocoyl Glycinate (2) | - | 3.25 |
| Coco-Glucoside (3) | 3.25 | 3.25 |
| PEG-150 pentaerythrityl tétrastéarate (4) | 1 | - |
| Acrylates/C10-30 Alkyl acrylate Crosspolymer (5) | - | 1 |
| PEG-7 glycéryl cocoate | - | 1 |
| Glycol distearate | - | 2 |
| Amidon de mais | | 2 |
| N-(2-hydroxyéthyl)-urée (6) | 2.5 | 10 |
| Triéthanolamine | - | 1 |
| Acide citrique | Qs pH7 | Qs pH7 |
| DMDM Hydantoin | 0.2 | 0.2 |
| Sodium Méthylparaben | 0.3 | 0.3 |
| Eau | Qsp100 | Qsp100 |

| | | |
|---|---|---|
| (1) Beaulight Shaa commercialisé par la société Sanyo (2) Amilite GCS12 commercialisé par la société Ajinomoto (3) Plantacare 818 UP commercialisé par la société Cognis (à 53% en matière active) (4) CROTHIX commercialisé par la société Croda. (5) Carbopol 1382 commercialisé par la société Noveon (6) Hydrovance commercialisé par la société National Starch (à 50% de matière active en solution aqueuse) | | |

## Revendications

1. Composition de nettoyage contenant dans un milieu aqueux, (1) au moins un tensioactif anionique choisi parmi les acides alkyl glycol carboxyliques et leurs sels, et (2) au moins un composé de formule (I) suivante : 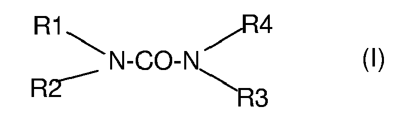 dans laquelle :
R1, R2, R3 et R4 représentent chacun, indépendamment l'un de l'autre, un atome d'hydrogène, un groupe alkyle en C1-C4 ou un groupe hydroxyalkyle en C2-C6 pouvant contenir de 1 à 5 groupes hydroxyles, où au moins un des radicaux R1 à R4 représente un groupe hydroxyalkyle,
ainsi que leurs sels, leurs solvats, et leurs isomères.

2. Composition selon la revendication 1, **caractérisée en ce qu'**elle comprend au moins 20 % en poids d'eau par rapport au poids total de la composition.

3. Composition selon la revendication 1 ou 2, **caractérisée en ce que**, dans les composés de formule (I), R1 désigne un groupe hydroxyalkyle en C2-C6 et R2, R3, R4 désignent, indépendamment l'un de l'autre, un atome d'hydrogène ou un groupe alkyle en C1-C4.

4. Composition selon l'une quelconque des revendications précédentes, **caractérisée en ce que**, dans les composés de formule (I), R1 désigne un groupe hydroxyalkyle en C2-C6 comprenant de 1 à 5 groupes hydroxyles, et R2, R3, R4 désignent un atome d'hydrogène.

5. Composition selon la revendication précédente, **caractérisée en ce que** R1 désigne un groupe hydroxyalkyle en C2-C6 comprenant 1 groupe hydroxyle.

6. Composition selon l'une quelconque des revendications précédentes, **caractérisée en ce que**, dans les composés de formule (I), R1 désigne un groupe hydroxyalkyle en C2-C4 comprenant 1 groupe hydroxyle et R2, R3, R4 désignent un atome d'hydrogène.

7. Composition selon l'une quelconque des revendications précédentes, **caractérisée en ce que** le composé de formule (I) est choisi parmi le N-(2-hydroxyéthyl)-urée ; le N-(2-hydroxypropyl)-urée ; le N-(3-hydroxypropyl)-urée ; le N-(2,3-dihydroxypropyl)-urée ; le N-(2,3,4,5,6-pentahydroxyhexyl)-urée ; le N-méthyl-N-(1,3,4,5,6-pentahydroxy-2-hexyl)-urée ; le N-méthyl-N'-(1-hydroxy-2-méthyl-2-propyl)-urée ; le N-(1-hydroxy-2-méthyl-2-propyl)-urée ; le N-(1,3-dihydroxy-2-propyl)-urée ; le N-(tris-hydroxyméthyl-méthyl)-urée; le N-éthyl-N'-(2-hydroxyéthyl)-urée ; le N,N-bis-(2-hydroxyéthyl)-urée ; le N,N'-bis-(2-hydroxyéthyl)-urée ; le N,N-bis-(2-hydroxypropyl)-urée ; le N,N'-Bis-(2-hydroxypropyl)-urée ; le N,N-Bis-(2-hydroxyéthyl)-N'-propyl-urée ; le N,N-Bis-(2-hydroxypropyl)-N'-(2-hydroxyéthyl)-urée ; le N-tert.Butyl-N'-(2-(hydroxyéthyl)-N'-(2-(hydroxypropyl)-urée; le N-(1,3-dihydroxy-2-propyl)-N'-(2-hydroxyéthyl)-urée ; le N,N-Bis-(2-hydroxyéthyl)-N',N'-dimethyl-urée ; le N,N,N',N'-tetrakis-(2-hydroxy-éthyl)-urée ; le N',N'-Bis-(2-hydroxyéthyl)-N',N'-bis-(2-hydroxypropyl)-urée ; et leurs mélanges.

8. Composition selon l'une quelconque des revendications précédentes, **caractérisée en ce que** le composé de formule (I) est le N-(2-hydroxyéthyl)-urée.

9. Composition selon l'une quelconque des revendications précédentes, **caractérisée en ce que** les sels des composés de formule (I) sont choisis parmi les sels de l'acide sulfurique, l'acide chlorhydrique, l'acide bromhydrique, l'acide iodhydrique, l'acide phosphorique, l'acide borique, l'acide propionique, l'acide acétique, l'acide téréphtalique, l'acide citrique, l'acide tartrique.

10. Composition selon l'une quelconque des revendications précédentes, **caractérisée en ce que** le composé de formule (I) est présent en une quantité allant de 0,1 à 50 % en poids par rapport au poids total de la composition.

11. Composition selon l'une quelconque des revendications précédentes, **caractérisée en ce que** le tensioactif anionique choisi parmi les acides alkyl glycol carboxyliques et leurs sels est un composé de formule (II) suivante :
R₁-CHOH-CH₂-O-COO⁻ X⁺ (II)
dans laquelle R₁ désigne un radical alkyle saturé ou insaturé, linéaire ou ramifié, comportant de 8 à 30 atomes de carbone, et X désigne l'hydrogène ou un cation minéral ou organique.

12. Composition selon la revendication précédente, **caractérisée en ce que** le tensioactif anionique est un composé de formule (II) dans laquelle R₁ désigne un radical alkyle saturé ou insaturé, linéaire ou ramifié comportant de 8 à 18 atomes de carbone.

13. Composition selon la revendication précédente, **caractérisée en ce que** le tensioactif anionique est le lauryl glycol carboxylate de sodium, ou sa forme acide.

14. Composition selon l'une quelconque des revendications précédentes, **caractérisée en ce que** la quantité de tensioactif(s) anionique(s) choisi(s) parmi les acides alkyl glycol carboxyliques et leurs sels va de 0,1 à 50 % en poids par rapport au poids total de la composition.

15. Composition selon l'une quelconque des revendications précédentes, **caractérisée en ce qu'**elle contient un ou plusieurs autres tensioactifs moussants, choisis parmi les tensioactifs non-ioniques, amphotères ou zwitterioniques, anioniques.

16. Composition selon l'une quelconque des revendications précédentes, **caractérisée en ce qu'**elle constitue un produit de nettoyage et/ou de démaquillage de la peau, un produit de douche, un shampoing, un après-shampoing, un produit de rasage, un masque à rincer, un produit exfoliant.

17. Utilisation cosmétique d'une composition cosmétique selon l'une quelconque des revendications 1 à 15, comme produit de nettoyage et/ou de démaquillage de la peau, comme produit de douche, comme shampoing ou après-shampoing, comme produit de rasage, comme masque à rincer, comme produit exfoliant.

18. Procédé de nettoyage d'une matière kératinique, **caractérisé en ce qu'**on applique sur la matière kératinique, une composition selon l'une quelconque des revendications 1 à 15 et qu'on la rince.
